(19) Europäisches Patentamt
European Patent Office
Office européen des brevets

(11) Numéro de publication: **0 017 602**
**B1**

(12) **FASCICULE DE BREVET EUROPEEN**

(45) Date de publication du fascicule du brevet: **20.10.82**

(51) Int. Cl.³: **C 07 D 309/38,** A 01 N 43/16

(21) Numéro de dépôt: **80420038.4**

(22) Date de dépôt: **26.03.80**

(54) **Dérivés de la phényl-2-pyrone-4, procédé pour leur préparation et leur utilisation comme fongicides.**

(30) Priorité: **28.03.79 FR 798569**

(43) Date de publication de la demande: **15.10.80 Bulletin 80/21**

(45) Mention de la délivrance du brevet: **20.10.82 Bulletin 82/42**

(84) Etats contractants désignés: **AT BE CH DE FR GB IT LU NL SE**

(56) Documents cités:
**US-A-4 060 533**

(73) Titulaire: **RHONE-POULENC AGROCHIMIE, 14-20, rue Pierre Baizet, F-69009 Lyon (FR)**

(72) Inventeur: **Guigues, François, 936 avenue Victor Hugo, F-69140 Rillieux (FR)**
Inventeur: **Trinh, Stéphane, 25 Boulevard de la *République, F-69410 - Champagne au Mont d'Or* (FR)**

(74) Mandataire: **Chaumette, Michel et al, RHONE-POULENC AGROCHIMIE BP 9163-09, F-69263 Lyon Cedex 1 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 017 602 B1

# Dérivés de la phényl-2-pyrone-4, procédé pour leur préparation et leur utilisation comme fongicides

La présente invention concerne de nouveaux dérivés de la phényl-2 pyrone-4, leur préparation et leur utilisation pour la protection des végétaux contre les maladies fongiques.

Les composés selon l'invention répondent à la formule générale (I)

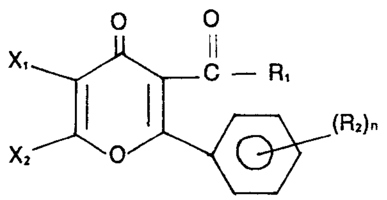

(composé I)

dans laquelle:

— $X_1$ et $X_2$ identiques ou différents représentent chacun un atome d'hydrogène ou d'halogène, sous réserve que l'un au moins de ces substituants représente un atome d'halogène.

— $R_1$ représente un radical alcoyloxyle contenant de 1 à 4 atomes de carbone, alcényloxyle contenant de 2 à 4 atomes de carbone, propargyloxyle, halogénoalcoyloxyle contenant de 1 à 4 atomes de carbone, amino, alcoylamino contenant de 1 à 4 atomes de carbone ou dialcoylamino dans lequel chacune des parties alcoyle, identiques ou différentes, contient de 1 à 4 atomes de carbone.

— $R_2$ représente un atome d'halogène, un radical alcoyle contenant de 1 à 5 atomes de carbone, alcoyloxyle contenant de 1 à 4 atomes de carbone, alcényloxyle contenant de 3 à 4 atomes de carbone, alcynyloxyle contenant de 3 à 4 atomes de carbone, alcoylthio contenant de 1 à 4 atomes de carbone, halogénoalcoyle contenant de 1 à 4 atomes de carbone, halogénoalcoyloxyle contenant de 1 à 4 atomes de carbone, halogénoalcoylthio contenant de 1 à 4 atomes de carbone, nitro, hydroxyle, cyano ou benzyloxyle,

— n est un nombre entier pouvant être égal à 0, 1, 2, 3, 4 ou 5, étant entendu que lorsque n est supérieur ou égal à 2, les substituants $R_2$ peuvent être identiques ou différents.

Certains dérivés de phényl-2-pyrone-4 ont déjà été décrits dans la littérature: ainsi le brevet US-A-4 060 533 revendique en tant que médicaments à propriétés antiallergiques des composés de formule:

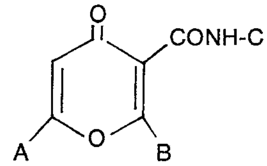

dans laquelle A et B réprésentent chacun un radical alcoyle inférieur ou un radical phényle éventuellement substitué par un atome d'halogène, ou un radical alcoyle ou alcoxy et C représente un radical alcoyle inférieur.

Les composés selon l'invention sont différents de ceux décrits par cette référence. Ils présentent des propriétés biologiques originales, et en particulier une action fongicide remarquable sur plusieurs espèces différentes de champignons, révélant de surcroît de surprenantes propriétés systémiques.

Une classe préférée des composés selon l'invention répond à la formule suivante:

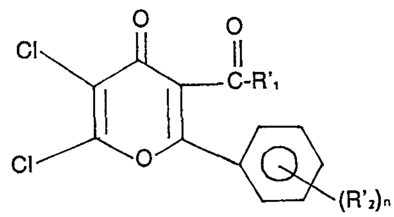

dans laquelle:

$R'_1$ représente un radical alcoyloxyle contenant de 1 à 4 atomes de carbone, allyloxyle, propargyloxyle, ou dialcoylamino dont chacune des parties alcoyles, identiques ou différentes contient de 1 à 4 atomes de carbone.

$R'_2$ représente un atome d'halogène, un radical alcoyle contenant de 1 à 4 atomes de carbone, alcoyloxyle, contenant de 1 à 4 atomes de carbone, allyloxyle, propargyloxyle, trifluorométhyle.

n' est un nombre entier égal à 1, 2 ou 3 étant entendu que lorsque n' est supérieur ou égal à 2, les radicaux $R'_2$ peuvent être identiques ou différents.

Les composés selon la formule I peuvent être préparés selon un procédé comportant les étapes successives suivantes:

## ETAPE A

Action d'un dérivé de l'acide benzoylacétique sur un alcoolate de magnésium, éventuellement préparé in situ, selon le schéma réactionnel:

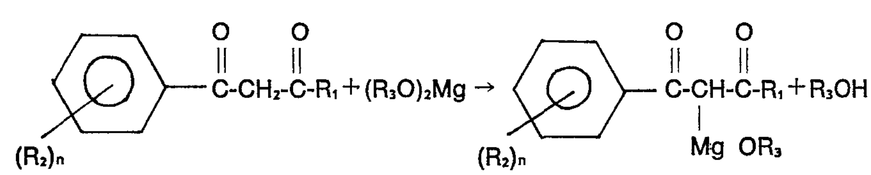

(composé II)

dans lequel $R_1$, $R_2$ et n ont la même signification que dans la formule I et $R_3$ représente un radical alcoyle contenant de 1 à 4 atomes de carbone.

La réaction s'effettue en milieu anhydre, avantageusement dans un solvant inerte (c'est à dire ne réagissant pas chimiquement avec les réactifs dans les conditions opératoires).

Les réactifs de départ sont d'abord mis en présence dans le solvant, puis le mélange réactionnel ainsi obtenu est chauffé à une température comprise entre 35 et 150°C environ, jusqu'à ce que la réaction soit terminée. Selon un mode de réalisation avantageux, le mélange réactionnel est chauffé à la température d'ébullition du solvant utilisé.

Comme solvant approprié, on peut citer des solvants organiques, polaires ou non polaires, tels que des hydrocarbures aromatiques (par exemple benzène, toluène ou xylènes), des hydrocarbures aliphatiques (par exemple hexane, heptane, cyclohexane), des éthers (par exemple l'éther diéthylique).

Comme alcoolate de magnésium, on utilise de préférence l'éthylate de magnésium que l'on peut préparer in situ en faisant réagir du magnésium sur de l'éthanol, dans un solvant organique inerte anhydre, en présence de tétrachlorure de carbone.

Le dérivé de l'acide benzoylacétique, matière de départ dans cette étappe A est préparé selon le procédé décrit dans Organic Syntheses - Vol. IV p. 415 pour la préparation du benzoylacétate d'éthyle.

ETAPE B

Action du chlorure d'un acide halogénoacrylique sur le dérivé alcoxymagnésien résultant de l'étape A (composé II), selon le schéma réactionnel suivant:

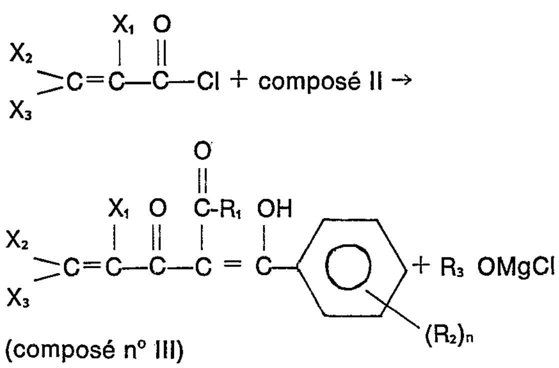


(composé n° III)

dans lequel $X_1$, $X_2$, $R_1$, $R_2$ et n ont la même signification que dans la formule I et $X_3$ représente un atome d'halogène, chlore de préférence.

La réaction s'effectue en milieu anhydre, avantageusement dans un solvant inerte, à une température comprise entre 0 et 40°C environ.

Comme solvants utilisables pour cette deuxième étape du procédé on peut citer ceux mentionnés plus haut en ce qui concerne l'étape A. Il est bien sûr avantageux d'utiliser un seul et même solvant pour ces deux étapes A et B.

ETAPE C

Cyclisation du composé III résultant de l'étape précédente pour donner le composé I selon le schéma réactionnel.

composé III

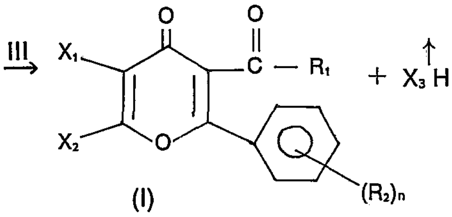

(I)

et décomposition par hydrolyse du composé de formule $R_3$ OMgCl résultant de l'étape précédente.

Cette cyclisation s'effectue en traitant tout d'abord le mélange réactionnel comprenant le composé III et le composé de formule $R_3OMgCl$, par une solution aqueuse diluée d'un acide fort, tel que l'acide sulfurique, à une température comprise entre 0 et 30°C environ (ce qui entraîne la décomposition par hydrolyse du composé de formule $R_3$ OMgCl), puis en chauffant la phase organique, préalablement séparée de la phase aqueuse, à une température comprise entre 30 et 160°C environ, éventuellement sous vide partiel, de manière à évaporer le solvant et à favoriser le départ de l'hydracide $X_3H$. Ce chauffage est prolongé jusqu'à ce que cesse le dégagement de l'hydracide.

Le composé de formule I ainsi obtenu est ensuite purifié par les méthodes usuelles telles que recristallisation dans un solvant approprié, chromatographie en phase liquide, etc.

Les exemples suivants, décrits à titre non limitatif illustrent la préparation des composés selon l'invention ainsi que leurs propriétés fongicides.

Tous les composés décrits dans ces exemples ont été identifiés par spectrométrie de résonance magnétique nucléaire (RMN). Les spectres ont été réalisés à 60 megahertz dans $CCl_4$ ou $CDCl_3$, avec l'hexaméthyldisiloxane comme référence interne.

Exemple 1

Préparation de la (méthyl-3-phényl)-2-ethoxycarbonyl-3 dichloro-5,6 pyrone-4 (composé n° 1).

5,7 g (0,05 mole) d'ethylate de magnésium et 10,3 g (0,05 mole) de m-methylbenzoylacétate d'éthyle sont dissous dans 100 ml de toluène, à température ambiante puis la solution ainsi obtenue est chauffée à reflux pendant une heure et enfin refroidie à 5°C par un bain d'eau glacée.

Dans cette solution maintenue à une température comprise entre 0 et 10°C, on coule goutte à goutte 9,7 g de chlorure de trichloro-acryloyle et le mélange réactionnel est maintenu sous agitation à température ambiante pendant 30 minutes.

Le mélange réactionnel est alors versé sur un mélange de 100 g de glace et de 10 ml d'acide sulfurique concentré. Après une heure d'agitation la phase toluénique est décantée et la phase aqueuse extraite par 50 ml de toluène.

Les phases toluéniques réunies sont lavées avec 100 ml d'eau puis avec 100 ml d'une solution de bicarbonate de sodium à 5% et enfin avec 100 ml d'eau. Après séchage sur sulfate de sodium anhydre et évaporation du toluène, on obtient un liquide huileux qui est ensuite chauffé à 80°C sous vide partiel de 16 mm Hg pendant 30 minutes, puis recristallisé dans 200 ml de methyl-cyclohexane.

On obtient ainsi 13 g de (méthyl-3-phényl)-2 éthoxycarbonyl-3 dichloro-5,6 pyrone-4 fondant à 113°C, soit un rendement de 79,5%.

Composition élémentaire

| | Calculée | Trouvée |
|---|---|---|
| C % | 55,04 | 55,08 |
| H % | 3,67 | 3,58 |
| Cl % | 21,71 | 21,39 |

Exemple 2

En opérant selon la méthode décrite dans l'exemple précédent, à partir des matières premières convenables, les composés n° 2 à 44 ont été préparés.

Les formules chimiques de ces composés, ainsi que leurs caractéristiques physicochimiques sont indiquées dans le tableau ci-après. Dans la colonne $R_2$ de ce tableau, le chiffre indiqué devant chacun des substituants $R_2$, signale la position du substituant $R_2$ concerné, sur le radical phényle (lui même en position-2 sur le cycle pyrone-4).

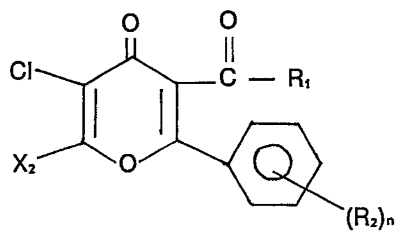

| Composé n° | $X_2$ | $R_1$ | $R_2$ | Formule brute | Point de fusion (°C) | Rendement % | Composition élémentaire | % calculé | % trouvé |
|---|---|---|---|---|---|---|---|---|---|
| 2 | Cl | -O $CH_3$ | 4-F | $C_{13}H_7Cl_2FO_4$ | 107,9 | 66 | C<br>H<br>Cl | 49,21<br>2,20<br>22,39 | 50,12<br>3,07<br>22,12 |
| 3 | Cl | -O $C_2H_5$ | 4-F | $C_{14}H_9Cl_2FO_4$ | 104,4 | 44 | C<br>H<br>Cl | 50,78<br>2,74<br>21,41 | 51,15<br>3,27<br>21,17 |
| 4 | Cl | -O $C_2H_5$ | 3-Cl | $C_{14}H_9Cl_3O_4$ | 115 | 78 | C<br>H<br>Cl | 48,34<br>2,59<br>30,64 | 48,35<br>2,60<br>30,36 |
| 5 | Cl | -O $C_2H_5$ | 4-$CH_3$ | $C_{15}H_{12}Cl_2O_4$ | 112,5 | 73 | C<br>H<br>Cl | 55,05<br>3,67<br>21,71 | 55,02<br>3,66<br>21,67 |
| 6 | Cl | -O $C_2H_5$ | 4-$C_2H_5$ | $C_{16}H_{14}Cl_2O_4$ | 70,5 | 71 | C<br>H<br>Cl | 56,30<br>4,11<br>20,82 | 56,36<br>4,24<br>20,46 |
| 7 | Cl | -O $C_2H_5$ | 4-CH $(CH_3)_2$ | $C_{17}H_{16}Cl_2O_4$ | 100,5 | 66,8 | C<br>H<br>Cl | 57,48<br>4,54<br>19,96 | 57,67<br>4,76<br>19,86 |
| 8 | Cl | -O $C_2H_5$ | 4-CH<($CH_2$-$CH_3$)($CH_3$) | $C_{18}H_{18}Cl_2O_4$ | 64 | 21 | C<br>H<br>Cl | 58,54<br>4,88<br>19,24 | 58,39<br>4,83<br>18,52 |
| 9 | Cl | -O $C_2H_5$ | 4-C $(CH_3)_3$ | $C_{18}H_{18}Cl_2O_4$ | 131,5 | 66 | C<br>H<br>Cl | 58,53<br>4,88<br>19,24 | 58,54<br>4,91<br>18,93 |
| 10 | Cl | -O $C_2H_5$ | 2-O $CH_3$ | $C_{15}H_{12}Cl_2O_5$ | 135 | 53 | C<br>H<br>Cl | 52,47<br>3,49<br>20,70 | 52,51<br>3,63<br>20,46 |

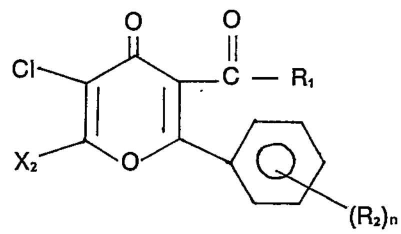

| Composé n° | $X_2$ | $R_1$ | $R_2$ | Formule brute | Point de fusion (°C) | Rendement % | Composition élémentaire | % calculé | % trouvé |
|---|---|---|---|---|---|---|---|---|---|
| 11 | Cl | $-O\ C_2H_5$ | $3-CF_3$ | $C_{15}H_9Cl_2F_3O_4$ | 113 | 67 | C<br>H<br>Cl | 47,24<br>2,36<br>18,37 | 46,43<br>2,42<br>18,59 |
| 12 | Cl | $-O\ C_2H_5$ | $3-CH_3$<br>4-F | $C_{15}H_{11}Cl_2FO_4$ | 124 | 70 | C<br>H<br>Cl | 52,17<br>3,18<br>20,58 | 52,12<br>3,23<br>20,66 |
| 13 | Cl | $-O\ C_2H_5$ | $3-CH\ (CH_3)_2$<br>4-F | $C_{17}H_{15}Cl_2FO_4$ | 116 | 65 | C<br>H<br>Cl | 54,79<br>4,02<br>19,03 | 54,59<br>4,16<br>19,10 |
| 14 | Cl | $-O\ C_2H_5$ | $3-CH_3$<br>$4-CH_3$ | $C_{16}H_{14}Cl_2O_4$ | 151,5 | 70 | C<br>H<br>Cl | 56,30<br>4,10<br>20,82 | 56,28<br>4,02<br>20,75 |
| 15 | Cl | $-O\ C_2H_5$ | $3-O\ CH_3$<br>$4-O\ CH_3$ | $C_{16}H_{14}Cl_2O_6$ | 132,5 | 68 | C<br>H<br>Cl | 51,47<br>3,75<br>19,03 | 51,58<br>3,85<br>19,12 |
| 16 | Cl | $-O\ C_2H_5$ | $3-CH_3$<br>$4-O\ CH_3$<br>$6-CH_3$ | $C_{17}H_{16}Cl_2O_5$ | 129 | 83 | C<br>H<br>Cl | 54,98<br>4,31<br>19,13 | 54,98<br>4,49<br>18,92 |
| 17 | Cl | $-O\ C_2H_5$ | $3-O\ CH_3$<br>$4-O\ CH_3$<br>$5-O\ CH_3$ | $C_{17}H_{16}Cl_2O_7$ | 131 | 57 | C<br>H<br>Cl | 50,62<br>3,97<br>17,60 | 50,57<br>3,86<br>17,35 |
| 18 | Cl | $-O(CH_2)_2-CH_3$ | $4-CH(CH_3)_2$ | $C_{18}H_{18}Cl_2O_4$ | 58,9 | 38 | C<br>H<br>Cl | 58,55<br>4,91<br>19,20 | 58,71<br>5,03<br>18,84 |
| 19 | Cl | $-O\ CH(CH_3)_2$ | $4-CH_3$ | $C_{16}H_{14}Cl_2O_4$ | 80 | 74 | C<br>H<br>Cl | 56,30<br>4,11<br>20,82 | 56,51<br>4,27<br>20,71 |
| 20 | Cl | $-O\ CH(CH_3)_2$ | $4-C(CH_3)_3$ | $C_{19}H_{20}Cl_2O_4$ | 106 | 70,5 | C<br>H<br>Cl | 59,53<br>5,22<br>18,54 | 59,66<br>5,33<br>18,28 |
| 21 | Cl | $-O\ CH(CH_3)_2$ | 4-F | $C_{15}H_{11}Cl_2FO_4$ | 97,5 | 81 | C<br>H<br>Cl | 52,20<br>3,20<br>20,68 | 52,90<br>3,07<br>20,65 |
| 22 | Cl | $-O\ CH_2-CH=CH_2$ | $4-C(CH_3)_3$ | $C_{19}H_{18}Cl_2O_4$ | 72,7 | 61,5 | C<br>H<br>Cl | 59,84<br>4,72<br>18,64 | 59,72<br>4,99<br>18,49 |
| 23 | Cl | $-O\ CH_2-C\equiv CH$ | 4-F | $C_{15}H_7Cl_2FO_4$ | 121,5 | 60 | C<br>H<br>Cl | 52,78<br>2,05<br>20,82 | 52,75<br>2,00<br>20,70 |
| 24 | Cl | $-O-CH_2-CH_2-Cl$ | 4-F | $C_{14}H_8Cl_3FO_4$ | 115,5 | 36,5 | C<br>H<br>Cl | 45,96<br>2,19<br>29,10 | 46,81<br>2,45<br>29,60 |
| 25 | Cl | $-N<^{C_2H_5}_{C_2H_5}$ | 4-F | $C_{16}H_{14}Cl_2FN\ O_3$ | 123,5 | 56 | C<br>H<br>N | 53,63<br>3,91<br>3,91 | 53,65<br>3,92<br>3,97 |

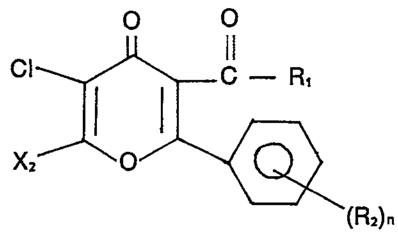

| Composé n° | $X_2$ | $R_1$ | $R_2$ | Formule brute | Point de fusion (°C) | Rendement % | Composition élémentaire | % calculé | % trouvé |
|---|---|---|---|---|---|---|---|---|---|
| 26 | H | -O $C_2H_5$ | 4-F | $C_{14}H_{10}ClFO_4$ | 85,0 | 40 | | | |
| 27 | Cl | O $C_2H_5$ | 3-$CH_3$ | $C_{15}H_{12}Cl_2O_4$ | 113 | 80,0 | C | 55,05 | 55,04 |
| | | | | | | | H | 3,67 | 3,55 |
| | | | | | | | Cl | 21,71 | 21,47 |
| | | | | | | | C | 53,50 | 53,74 |
| 28 | Cl | 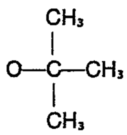 | 4-F | $C_{16}H_{13}Cl_2FO_4$ | 109 | 50,1 | H | 3,60 | 3,73 |
| | | | | | | | Cl | 19,80 | 19,74 |
| 29 | Cl | $OC_2H_5$ | 3-Cl | $C_{14}H_8Cl_3FO_4$ | 140,3 | 63,9 | C | 45,96 | 46,08 |
| | | | 4-F | | | | H | 2,19 | 2,22 |
| | | | | | | | Cl | 29,14 | 28,95 |
| 30 | Cl | O $C_2H_5$ | 2-$CF_3$ | $C_{15}H_9Cl_2F_3O_4$ | 82,5 | 65,6 | C | 47,24 | 47,12 |
| | | | | | | | H | 2,36 | 2,50 |
| | | | | | | | Cl | 18,64 | 17,84 |
| 31 | Cl | $OC_2H_5$ | 2-Cl | $C_{14}H_9Cl_3O_4$ | 126,8 | 58,5 | C | 48,34 | 47,82 |
| | | | | | | | H | 2,59 | 2,68 |
| | | | | | | | Cl | 30,64 | 30,33 |
| 32 | Cl | $OC_2H_5$ | 4-Cl | $C_{14}H_9Cl_3O_4$ | 111,6 | 48,9 | C | 48,34 | 48,60 |
| | | | | | | | H | 2,59 | 2,76 |
| | | | | | | | Cl | 30,65 | 30,22 |
| 33 | Cl | $OC_2H_5$ | 3-Br | $C_{14}H_9BrCl_2O_4$ | 120,5 | 68,0 | C | 42,86 | 42,52 |
| | | | | | | | H | 2,29 | 2,28 |
| | | | | | | | Br | 20,41 | 19,93 |
| | | | | | | | Cl | 18,11 | 17,39 |
| 34 | Cl | $OC_2H_5$ | 4-Br | $C_{14}H_9BrCl_2O_4$ | 112,7 | 66,3 | C | 42,86 | 43,37 |
| | | | | | | | H | 2,29 | 2,55 |
| | | | | | | | Br | 20,41 | 19,70 |
| | | | | | | | Cl | 18,11 | 18,08 |
| 35 | Cl | $OC_2H_5$ | 3-F | $C_{14}H_9Cl_2O_4$ | 104,0 | 67,3 | C | 50,75 | 50,92 |
| | | | | | | | H | 2,72 | 2,72 |
| | | | | | | | Cl | 21,45 | 21,38 |
| 36 | Cl | $OC_2H_5$ | 3-Cl | $C_{14}H_8Cl_4O_4$ | 128,1 | 70,7 | C | 43,98 | 44,36 |
| | | | 5-Cl | | | | H | 2,09 | 2,54 |
| | | | | | | | Cl | 37,17 | 36,68 |
| 37 | Cl | $OC_2H_5$ | 2-Cl | $C_{14}H_8Cl_4O_4$ | 92,5 | 57,6 | C | 43,98 | 43,85 |
| | | | 4-Cl | | | | H | 2,09 | 2,23 |
| | | | | | | | Cl | 37,17 | 36,43 |
| 38 | Cl | $OC_2H_5$ | 2-Cl | $C_{14}H_7Cl_5O_c$ | 176,4 | 44,3 | C | 40,34 | 40,82 |
| | | | 3-Cl | | | | H | 1,68 | 2,00 |
| | | | 5-Cl | | | | Cl | 42,62 | 41,90 |
| 39 | Cl | $OC_2H_5$ | 4-$NO_2$ | $C_{14}H_9Cl_2NO_6$ | 123,6 | 49,5 | C | 46,93 | 46,27 |
| | | | | | | | H | 2,51 | 2,48 |
| | | | | | | | N | 3,91 | 3,95 |
| | | | | | | | Cl | 19,83 | 19,93 |

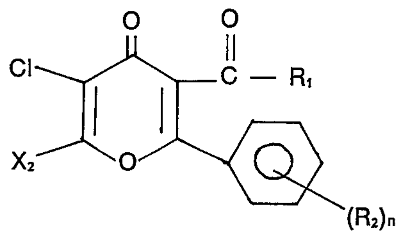

| Composé n° | $X_2$ | $R_1$ | $R_2$ | Formule brute | Point de fusion (°C) | Rendement % | Composition élémentaire | % calculé | % trouvé |
|---|---|---|---|---|---|---|---|---|---|
| 40 | Cl | $OC_2H_5$ | 4-$CF_3$ | $C_{15}H_9Cl_2F_3O_4$ | 68,5 | 44,6 | C | 47,24 | 47,39 |
| | | | | | | | H | 2,36 | 2,26 |
| | | | | | | | Cl | 18,63 | 18,77 |
| 41 | Cl | $OC_2H_5$ | 4-$OC_2H_5$ | $C_{16}H_{14}Cl_2O_5$ | 115,5 | 51,0 | C | 53,78 | 53,59 |
| | | | | | | | H | 3,92 | 3,82 |
| | | | | | | | Cl | 19,89 | 19,69 |
| 42 | Cl | $OC_2H_5$ | 4-O-$CH_2$- 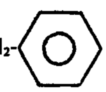 | $C_{21}H_{16}Cl_2O_5$ | 119,8 | 47,0 | C | 60,14 | 59,97 |
| | | | | | | | H | 3,82 | 3,78 |
| | | | | | | | Cl | 16,94 | 16,39 |
| 43 | Cl | $OC_2H_5$ | 4-$OCH_2CH=CH_2$ | $C_{17}H_{14}Cl_2O_5$ | 108,8 | 35,8 | C | 55,28 | 56,01 |
| | | | | | | | H | 3,79 | 3,76 |
| | | | | | | | Cl | 19,24 | 19,41 |
| 44 | Cl | $OC_2H_5$ | 4-$OCH_2$-$C{=}CH$ | $C_{17}H_{12}Cl_2O_5$ | 95,6 | 23,3 | C | 55,58 | 55,03 |
| | | | | | | | H | 3,26 | 3,18 |
| | | | | | | | Cl | 19,35 | 18,56 |

Exemple 3

Test in vitro de l'activité antifongique des composés selon l'invention. On étudie l'action des composés selon l'invention sur le champignon suivant: Piricularia oryzae, responsable de la piriculariose du riz.

Pour chaque essai, on opère de la manière suivante: dans des tubes à essai, on place 5 ml de gélose (milieu maltagar), puis on bouche chaque tube et on le stérilise 20 minutes à 120°C. Les tubes sont ensuite placés dans un bain-marie maintenu à 60°C. Ensuite, à l'aide d'une pipette, on injecte dans chaque tube une quantité déterminée d'une solution acétonique à 1% du composé à tester, de façon à obtenir dans la culture une concentration déterminée de ce composé. Au bout de 24 heures, les tubes sont ensemencés par injection à la seringue de 0,5 ml de suspensions de spores contenant environ 100 000 spores/ml. On prend comme témoin, un tube analogue au précédent, mais dont le milieu gélosé ne contient pas de matière active.

Les tubes sont conservés pendant 7 jours, à 26°C, à l'obscurité et on compare alors la croissance du champignon dans les tubes contenant la matière active à tester, à celle du témoin non traité. On détermine ainsi, pour chacun des composés testés, la plus faible dose permettant d'inhiber totalement le développement du champignon considéré.

Cette dose est respectivement de:

0,2 g/l pour les composés n° 14, 15, 18, 26 et 38
0,1 g/l pour les composés n° 39, 41 et 42
0,05 g/l pour les composés n° 1, 3, 6, 9, 10, 11, 12, 17, 19, 27, 28, 29, 31, 32, 33, 34, 36, 37, 40

Elle est égale ou inférieure à 0,01 g/l pour les composés n° 2, 4, 5, 7, 8, 13, 16, 20, 21, 22, 23, 30, 35, 43 et 44.

Exemple 4

Test in vivo sur Plasmopara viticola, sur plants de vigne (traitement préventif).

On traite par pulvérisation au pistolet, des plants de vigne (cépage Gamay) cultivés en pots, sur la face inférieure des feuilles, avec une suspension aqueuse d'une poudre mouillable ayant la composition pondérale suivante:

— matière active à tester 20%
— défloculant (lignosulfonate de sodium) 5%
— mouillant (alcoylarylsulfonate de sodium) 1%
— charge (silicate d'aluminium) 74%

à la dilution voulue, contenant la matière active à tester à la dose considérée. Chaque test fait l'objet de trois répétitions.

Au bout de 48 heures, la contamination est effectuée par pulvérisation, sur la face inférieure des feuilles d'une suspension aqueuse de 80.000 unités/cm³ environ de spores de Plasmopara viticola, responsable du mildiou de la vigne. Ensuite, les pots sont placés pendant 48 heures en cellule d'incubation à 100% d'humidité relative et à 20°C.

On effectue le contrôle des plants 9 jours après l'infestation.

Dans ces conditions, on observe qu'une protection totale (>95%) est obtenue aux doses suivantes dans le cas des composés cités ci-après:

— 0,015 g/l: composé n° 34
— 0,06 g/l: composés n° 1 et 35
— 0,12 g/l: composés n° 31 et 41
— 0,25 g/l: composés n° 11, 18, 23, 33, 36, 37, 38, 39, 40.

A cette même dose de 0,25 g/l, les composés n° 2, 3, 4, 5, 10, 12, 13, 15, 16 et 17 exercent une bonne protection (de 75 à 95%). Par ailleurs, dans cet essai, aucun phénomène de phytotoxicité n'a été observé.

Exemple 5

Test in vivo sur «Uromyces phaseoli», responsable de la rouille du haricot (traitement préventif).

Des plants de haricots sont cultivés, dans des pots de 8 cm de diamètre, remplis de tourbe. Au stade deux feuilles cotylédonaires, les plants sont traités par pulvérisation avec une suspension aqueuse de la même poudre mouillable qu'à l'exemple 4, contenant le produit à tester, à la dose voulue.

Au bout de 48 heures, on pulvérise les haricots avec une suspension de spores (50.000 sp/$cm^3$) obtenue à partir de plants contaminés. On place ensuite les haricots d'abord en cellule d'incubation à 100% d'humidité relative et 20°C, pendant 48 H, puis en serre dans les conditions suivantes:

| | |
|---|---|
| — Température jour | 20°C - 25°C |
| — Température nuit | 15°C - 20°C |
| — Humidité relative | 70 à 80% |

Le contrôle se fait le 15ème jour après la contamination, par comparaison avec le témoin non traité.

Dans ces conditions, on observe que, à la dose de 0,50 g/l les composés n° 11, 27, 30, 31, 35, 36 et 40 exercent une protection totale (> 95%) et les composés n° 3 et 4 une bonne protection (de 75 à 95%).

Exemple 6

Test in vivo sur «Puccinia striiformis» responsable de la rouille jaune des céréales (traitement préventif).

Des grains de blé, variété «Joss» sont semés dans des pots de 8 cm de diamètre. Huit jours après le semis, les plants de blé sont traités par pulvérisation au moyen d'une suspension aqueuse d'une poudre mouillable, ayant la composition pondérale suivante:

| | |
|---|---|
| (fluoro-4-phenyl)-2ethoxycarbonyl-3 dichloro-5,6 pyrone-4 (composé n° 3) | 20% |
| — Lignosulfonate de calcium | 5% |
| — Alcoylarylsulfonate | 1% |
| — Silicate d'aluminium | 74% |

Le traitement est effectué pour différentes concentrations en matière active de la suspension acqueuse, chaque test faisant l'objet de trois répétitions.

Au bout de 48 heures, après le traitement, on pulvérise le blé avec une suspension de spores (50.000 spores/ml) obtenue à partir de plants contaminés. On place ensuite le blé en cellule d'incubation réglée comme à l'exemple 5. Le contrôle se fait le 15ème jour après la contamination, par comparaison avec un témoin non traité. Dans ces conditions, on observe, qu'aux doses de 0,5 g/l, 0,250 g/l et 0,125 g/l, le composé n° 3 assure une bonne protection (de 75 à 95%). Au cours de cet essai, aucun phénomène de phytotoxicité vis-à-vis des plantes traitées n'a été observé.

Exemple 7

Test de plein champ sur «Puccinia recondita» responsable de la rouille brune des céréales.

Des grains de blé, de variété «Nebraska» sont semés à l'automne dans des parcelles de 50 $m^2$ chacune. Le 10 mai de l'année suivante, les plants de blé sont traités par pulvérisation au moyen d'une bouillie obtenu en diluant par de l'eau une poudre mouillable ayant la composition pondérale suivante:

| | |
|---|---|
| — matière active à tester | 500 |
| — mouillant (alcoylarylsulfonate de sodium) | 10 |
| — défloculant (lignosulfonate de calcium) | 50 |
| — silice antimottante | 50 |
| — charge (kaolinite) | 390 |

Cette bouillie contient 200 g/hl de matière active et est appliquée par pulvérisation à raison de 500 l/ha ce qui correspond à une dose de 1000 g/ha de matière active, chaque test faisant l'objet de 4 répétitions. On observe vers la fin de mai l'apparition d'une contamination par rouille brune. Un deuxième traitement est effectué le 7 juin en utilisant pour chacune des parcelles les mêmes conditions que pour celui du 10 mai.

Le contrôle est effectué le 12 juillet, on évalue alors (en %) la surface foliaire recouverte par des pustules de rouille brune et on compare aux résultats observés dans le cas d'une parcelle témoin non traitée: dans ces conditions, on observe que ce % est de 1,9% en moyenne, dans le cas des parcelles traitées par la composition contenant le composé n° 3 et de 35,7% dans les parcelles témoins.

Exemple 8

Test de plein champ sur «Venturia inequalis» responsable de la tavelure du pommier.

Des parcelles de 30 m² chacune, comportant par parcelle 5 pommiers de variété «golden delicious» sont traitées le 15 mars, au moment de l'apparition de la maladie, par une bouillie obtenue en diluant par de l'eau la même composition qu'à l'exemple 7. Cette bouillie contient 100 g/hl de matière active (composé n° 3) et est appliquée par pulvérisation à raison de 1.000 l/ha ce qui correspond à une dose de 1 Kg/ha de matière active.

D'autres parcelles sont traitées à la même date par une bouillie contenant 150 g/l de captane et appliquée à raison de 1000 l/ha, ce qui correspond à 1,5 Kg/ha de captane. Ces traitements sont répétés, selon les mêmes conditions, tous les 15 jours, soit au total 6 traitements pour chacune des matières actives appliquées. Chaque test fait l'objet de 4 répétitions. Des parcelles sont laissées sans traitement comme témoin.

Le contrôle est effectué le 20 juin et on détermine alors le % de feuilles malades et le % de la surface des feuilles qui a été contaminé.

| | dose Kg/ha | Nombre de traitements | % de feuilles malades | % de la surface contaminée |
|---|---|---|---|---|
| composé No 3 | 1 | 6 | 0,3 | 0,01 |
| captane | 1,5 | 6 | 1,3 | 0,1 |
| témoin | 0 | — | 69,8 | 18,6 |

Exemple 9

Test de plein champ sur «Septoria apii» responsable de la septoriose du céléri.

Des plants de céléri, de variété «Gennevilliers» sont repiqués le 6 juin dans des parcelles de 2 m² dans chacune desquelles on repique le 1er juillet un plant contaminé par la maladie.

Des traitements sont effectués le 7 août au moyen d'une bouillie préparée en diluant par de l'eau la même composition qu'à l'exemple 7. Cette bouillie contient 100 g/hl de matière active et est appliquées par pulvérisation à raison de 1000 l/ha, ce qui correspond à une dose de 1 kg/ha de matière active.

D'autres parcelles sont traitées aux mêmes dates par du manèbe à raison de 2,4 kg/ha de matière active à chaque traitement. Des parcelles sont laissées sans traitement, comme témoin.

Le contrôle est effectué le 29 août et on détermine alors le % de la surface des feuilles qui a été contaminé.

| | dose kg/ha | nombre de traitements | % de la surface contaminée |
|---|---|---|---|
| composé No 3 | 1 | 2 | 19,1 |
| manèbe | 2,4 | 2 | 24,0 |
| témoin | | | 60,0 |

Les exemples précédents illustrent clairement les remarquables propriétés antifongiques des composés selon l'invention, sur différentes espèces de champignons telles que les basidiomycètes (notamment Puccinia striiformis, Puccinia recondita, Uromycès phaseoli, les ascomycètes (notamment Venturia inequalis), les fungi imperfecti (notamment Piricularia orizae, Septoria apii), les phycomycètes (notamment Plasmopara viticola) ainsi que leur absence de phytotoxicité vis-à-vis des cultures considérées.

D'excellents résultats ont également été observés vis-à-vis de plusieurs champignons de semences, tels que Fusarium nivale et Fusarium culmorum.

Des résultats particulièrement avantageux ont été observés dans le cas des composés suivants:

-(fluoro-4 phényl)-2 éthoxycarbonyl-3 dichloro-5,6 pyrone-4 (composé n° 3)

-(trifluoromethyl-3 phényl)-2 éthoxycarbonyl-3 dichloro-5,6 pyrone-4 (composé n° 11).

Les doses d'emploi peuvent varier dans de larges limites selon la virulence du champignon et les conditions climatiques. D'une manière générale des compositions contenant de 0,01 à 5 g/l de matière active conviennent bien.

Pour leur emploi dans la pratique, les composés selon l'invention sont rarement utilisés seuls. Le plus souvent, ils font partie de compositions qui sont également comprises dans le cadre de l'invention, et qui comprennent en général, en plus de la matière active un support et éventuellement un agent tensioactif. La teneur en matière active de ces compositions est généralement comprise entre 0,0005 et 95% en poids.

Au sens de la présente description, le terme «support» désigne une matière organique ou minérale, naturelle ou synthétique, avec laquelle la matière active est associée pour faciliter son application sur la plante, sur des graines ou sur le sol, ou son transport, ou sa manipulation. Ce support doit donc être inerte et acceptable en agriculture, notamment par la plante. Le support peut être solide (argiles, silicates naturels ou synthétiques, résines, cires, engrais solides...) ou liquide (eau, alcools, cétones, fraction de pétrole, hydrocarbures chlorés, hydrocarbures aromatiques ou paraffiniques, gaz liquéfiés etc.).

L'agent tensioactif peut être un agent émulsionnant, dispersant ou mouillant, de type ionique ou non ionique. On peut citer par exemple, des sels d'acides polyacryliques, des sels d'acides lignosulfoniques, des sels d'acides phénolsulfoniques ou naphthalènesulfoniques, des polycondensats d'oxyde d'éthylène sur des alcools gras, ou sur des acides gras, ou sur des amines grasses, des phénols substitués (notamment des alkylphénols ou des arylphénols), des sels d'esters d'acides sulfosucciniques, des esters phosphoriques d'alcools ou de phénols polyoxyethylés.

Ces compositions peuvent également contenir toute sorte d'autres ingrédients tels que par exemple des colloïdes protecteurs, des adhésifs, des épaississants, des agents de pénétration, des stabilisants, des sequestrants etc., ainsi que d'autres matières actives connues à propriétés pesticides (notamment insecticides ou fongicides) ou favorisant la croissance des plantes (notamment des engrais).

Les compositions selon l'invention peuvent être préparées sous la forme de poudres mouillables, de poudres solubles, de poudres pour poudrages, de solutions, de concentrés émulsionnables, d'émulsions, de concentrés en suspension, d'aérosols.

Les poudres mouillables sont habituellement préparées de manière qu'elles contiennent de 20 à 95% en poids de matière active et elles contiennent habituellement en plus du support solide, de 0 à 5% en poids d'un agent mouillant, de 3 à 10% en poids d'un agent dispersant, et, quand c'est nécessaire, de 0 à 10% en poids d'un ou de stabilisants et/ou d'autres additifs, comme des agents de pénétration, des adhésifs ou des agents antimottants, colorants, etc.

A titre d'exemple, voici la composition pondérale d'une poudre mouillable selon l'invention:

- matière active (composé n° 3) 500 g
- lignosulfonate de calcium (défloculant) 50 g
- isopropylnaphthalène sulfonate (agent mouillant anionique) 10 g
- silice antimottante 50 g
- kaolin 390 g

Un autre exemple de poudre mouillable selon l'invention à la composition pondérale suivante:

- matière active (composé n° 1) 700 g
- dibutylnaphthylsulfonate de sodium 50 g
- produit de condensation en proportions 3/2/1 d'acide naphthalène sulfonique, d'acide phenolsulfonique et de formaldéhyde 30 g
- kaolin 100 g
- craie de Champagne 120 g

Un troisième exemple de poudre mouillable selon l'invention à la composition pondérale suivante:

- matière active 250 g
- lignosulfonate de calcium 45 g
- mélange équipondéral de craie de Champagne et d'hydroxyéthylcellulose 19 g
- dibutylnaphthalène sulfonate de sodium 15 g
- silice 195 g
- craie de Champagne 195 g
- kaolin 281 g

Les composés selon l'invention peuvent également être utilisés sous forme de poudres pour poudrage, ayant, par exemple la composition pondérale suivante:

- matière active 50 g
- talc 950 g

Les concentrés émulsionnables, applicables en pulvérisation contiennent habituellement de 10 à 50% en poids/volume de matière active et de 2 à 20% d'un agent émulsifiant.

Ils peuvent également contenir, si nécessaire, de 2 à 20% en poids/volume d'additifs appropriés tels que des agents tensioactifs, des stabilisants, des agents de pénétration, des inhibiteurs de corrosion, des colorants, des adhésifs.

A titre d'exemple, voici la composition d'un concentré émulsionnable, les quantités étant exprimées en g/litre:

- matière active (composé n° 3) 400 g/l
- dodécylbenzène sulfonate alcalin 24 g/l
- nonylphénol oxyéthylé à 10 molécules d'oxyde d'éthylène 16 g/l
- cyclohexanone 200 g/l
- solvant aromatique q.s.p. 1 litre

Les concentrés en suspension, également applicables en pulvérisation, sont préparés de manière que l'on obtienne un produit fluide stable ne se déposant pas et ils contiennent habituellement de 10 à 75% en poids de matière active, de 0,5 à 15% en poids d'agents tensioactifs, de 0,1 à 10% en poids d'agents thixotropes, de 0 à 10% d'additifs appropriés, comme des antimousses, des inhibiteurs de corrosion, des stabilisants, des agents de pénétration et des adhésifs et comme support, de l'eau ou un liquide organique dans lequel la matière active est sensiblement insoluble: certaines matières solides organiques ou des sels minéraux peuvent être dis-

souts dans le support pour aider à empêcher la sédimentation ou comme antigels pour l'eau.

Les dispersions et émulsions aqueuses obtenues en diluant par de l'eau des compositions mentionnées plus haut, notamment les poudres mouillables et concentrés émulsionnables selon l'invention, sont également comprises dans le cadre général de la présente invention. Les émulsions ainsi obtenues peuvent être du type eau-dans-l'huile ou du type huile-dans-l'eau et elles peuvent avoir une consistance épaisse comme celle d'une mayonnaise.

**Revendications pour les Etats contractants:**
BE, CH, DE, FR, GB, IT, LU, NL, SE

1. Dérivé de la phényl-2-pyrone-4 caractérisé en ce qu'il répond à la formule

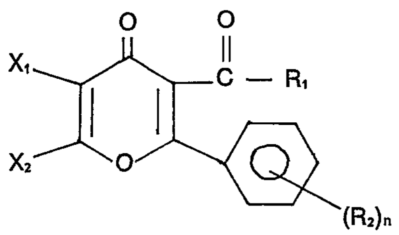

dans laquelle:

— $X_1$ et $X_2$, identiques ou différents représentent chacun un atome d'hydrogène ou d'halogène, sous réserve que l'un au moins de ces substituants représente un atome d'halogène.

— $R_1$ représente un radical alcoyloxyle contenant de 1 à 4 atomes de carbone, alcényloxyle contenant de 2 à 4 atomes de carbone, le radical propargyloxyle, un radical halogénoalcoyloxyle contenant de 1 à 4 atomes de carbone, amino, alcoylamino contenant de 1 à 4 atomes de carbone, dialcoylamino dans lequel les parties alcoyle ($C_1$-$C_2$) sont identiques ou différentes.

— $R_2$ représente un atome d'halogène, un radical alcoyle contenant de 1 à 5 atomes de carbone, alcoyloxyle contenant de 1 à 4 atomes de carbone, alcényloxyle contenant de 3 à 4 atomes de carbone, alcynyloxyle contenant de 3 à 4 atomes de carbone, alcoylthio contenant de 1 à 4 atomes de carbone, halogénoalcoyle contenant de 1 à 4 atomes de carbone, halogénoalcoyloxyle contenant de 1 à 4 atomes de carbone, halogénoalcoylthio contenant de 1 à 4 atomes de carbone, nitro, hydroxyle, cyano ou benzyloxyle.

— n est un nombre entier pouvant être égal à 0, 1, 2, 3, 4 ou 5, étant entendu que lorsque n est supérieur ou égal à 2, les substituants $R_2$ peuvent être identiques ou différents.

2. Composé selon la revendication 1 caractérisé en ce qu'il répond à la formule.

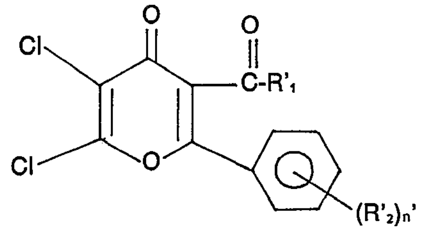

dans laquelle:

— $R'_1$ représente un radical alcoyloxyle contenant de 1 à 4 atomes de carbone, allyloxyle, propargyloxyle, dialcoylamino dans lequel chacune des parties alcoyles, identiques ou différentes, contient de 1 à 4 atomes de carbone.

— $R'_2$ représente un atome d'halogène, un radical alcoyle contenant de 1 à 4 atomes de carbone, alcoyloxyle contenant de 1 à 4 atomes de carbone, allyloxyle, propargylyloxyle ou le radical trifluorométhyle.

— n' est un nombre entier égal à 1, 2 ou 3, étant entendu que lorsque n' est supérieur ou égal à 2, les radicaux $R'_2$ peuvent être identiques ou différents.

3. Composé selon la revendication 1 caractérisé en ce que ce composé est le (fluoro-4-phényl)-2-éthoxycarbonyl-3 dichloro-5,6 pyrone-4.

4. Composé selon la revendication 1 caractérisé en ce que ce composé est le (trifluoromethyl-3-phenyl)-2-éthoxycarbonyl-3-dichloro-5,6 pyrone-4.

5. Procédé de préparation des composés selon la revendication 1 caractérisé en ce qu'il comprend les trois étapes suivantes:

A - Action d'un dérivé de l'acide benzoylacétique sur un alcoolate de magnésium, éventuellement préparé in situ, selon le schéma réactionnel.

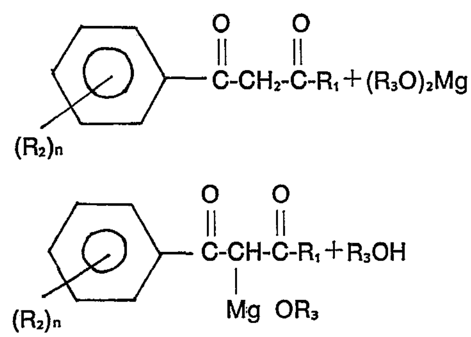

dans lequel $R_1$, $R_2$ et n ont la même signification que dans la revendication 1 et $R_3$ représente un radical alcoyle contenant de 1 à 4 atomes de carbone.

B - Action du chlorure d'un acide trihalogénoacrylique sur le dérivé alcoxymagnésien résultant de l'étape A, selon le schéma réactionnel

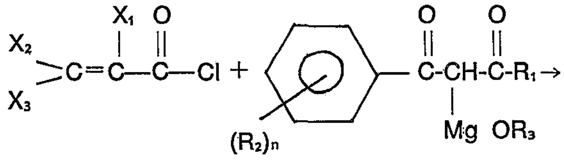

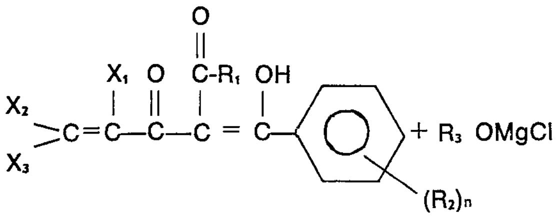


dans lequel $X_1$, $X_2$, $R_1$, $R_2$ et n ont la même signification que dans la revendication 1 et $X_3$ représente un atome d'halogène.

C - Cyclisation du composé énolique résultant de l'étape B, pour donner le composé selon la revendication 1.

6. Procédé selon la revendication 5 caractérisé en ce que l'étape A est effectuée en milieu anhydre, dans un solvant inerte à une température comprise entre 35 et 150°C.

7. Procédé selon la revendication 5 caractérisé en ce que l'étape B est effectuée en milieu anhydre, dans un solvant inerte, à une température comprise entre 0 et 40°C.

8. Procédé selon la revendication 5 caractérisé en ce que l'étape C est effectuée en traitant par une solution aqueuse diluée d'un acide fort, le mélange réactionnel résultant de l'étape B, à une température comprise entre 0 et 30°C, puis en séparant la phase organique de la phase aqueuse, et en chauffant ensuite la phase organique à une température comprise entre 30° et 160°C.

9. Composition utilisable pour la protection des végétaux contre les maladies fongiques, caractérisé en ce qu'elle contient une quantité efficace d'au moins un composé selon la revendication 1, en association avec un support et/ou un agent tensioactif utilisables en agriculture et compatibles avec ladite matière active.

10. Composition selon la revendication 9, caractérisé en ce qu'elle contient de 0,01 à 95% en poids de matière active.

11. Procédé de traitement des plantes contre les maladies fongiques, caractérisé en ce que l'on utilise une composition selon l'une des revendications 9 à 10.

**Revendications pour l'Etat contractant:** AT

1. Composition fongicide, pour la lutte contre les maladies fongiques des plantes, caractérisée en ce qu'elle contient comme matière active au moins un composé répondant à la formule générale:

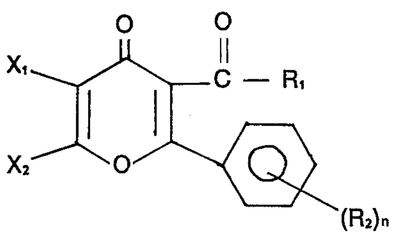

dans laquelle:

— $X_1$ et $X_2$, identiques ou différents, représentent chacun un atome d'hydrogène ou d'halogène, sous réserve que l'un au moins de ces substituants représente un atome d'halogène.

— $R_1$ représente un radical alcoyloxyle contenant de 1 à 4 atomes de carbone, alcényloxyle contenant de 2 à 4 atomes de carbone, le radical propargyloxyle, un radical halogénoalcoyloxyle contenant de 1 à 4 atomes de carbone, amino, alcoylamino contenant de 1 à 4 atomes de carbone, dialcoylamino dans lequel les parties alcoyle ($C_1$-$C_4$) sont identiques ou différentes.

— $R_2$ représente un atome d'halogène, un radical alcoyle contenant de 1 à 5 atomes de carbone, alcoyloxyle contenant de 1 à 4 atomes de carbone, alcényloxyle contenant de 3 à 4 atomes de carbone, alcynyloxyle contenant de 3 à 4 atomes de carbone, alcoylthio contenant de 1 à 4 atomes de carbone, halogénoalcoyle contenant de 1 à 4 atomes de carbone, halogénalcoyloxyle contenant de 1 à 4 atomes de carbone, halogénoalcoylthio contenant de 1 à 4 atomes de carbone, nitro, hydroxyle, cyano ou benzyloxyle.

— n est un nombre entier pouvant être égal à 0, 1, 2, 3, 4 ou 5, étant entendu que lorsque n est supérieur ou égal à 2, les substituants $R_2$ peuvent être indentiques ou différents.

2. Composition selon la revendication 1, caractérisée en ce qu'elle contient comme matière active au moins un composé répondant à la formule:

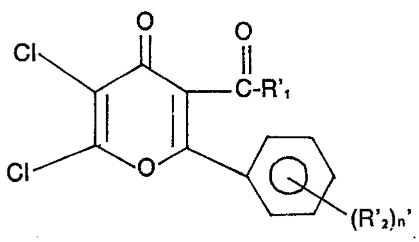

dans laquelle:

— $R'_1$ représente un radical alcoyloxyle contenant de 1 à 4 atomes de carbone, allyloxyle, propargyloxyle, dialcoylamino dans lequel chacune des parties alcoyle, identiques ou différentes, contient de 1 à 4 atomes de carbone.

— $R'_2$ représente un atome d'halogène, un radical alcoyle contenant de 1 à 4 atomes de carbone, alcoyloxyle contenant de 1 à 4 atomes de carbone, allyloxyle, propargyloxyle ou le radical trifluorométhyle.

— n' est un nombre entier égal à 1, 2 ou 3, étant entendu que lorsque n' est supérieur ou égal à 2, les radicaux $R'_2$ peuvent être identiques ou différents.

3. Composition selon la revendication 1, caractérisée en ce qu'elle contient comme matière active au moins un composé choisi parmi le (fluoro-4-phényl)-2 éthoxycarbonyl-3 dichloro-5,6 pyrone-4 et le (trifluorométhyl-3-phényl)-2 éthoxycarbonyl-3-dichloro-5,6 pyrone-4.

4. Composition selon la revendication 1, caractérisée en ce qu'elle contient de 0,01 à 95% en poids de matière active en association avec un support et/ou un agent tensioactif utilisables en agriculture.

5. Procédé de traitement des plantes contre les maladies fongiques, caractérisée en ce que l'on utilise une composition selon l'une des revendications 1 à 4.

6. Procédé de préparation d'un composé de formule générale:

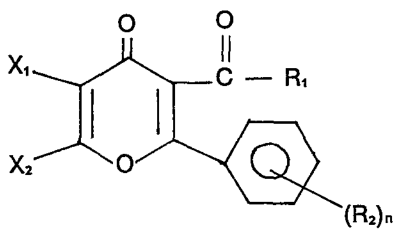

dans laquelle $X_1$, $X_2$, $R_1$, $R_2$ et n ont la même signification que dans la revendication 1, caractérisé en ce qu'il comprend les trois étapes suivantes:

A - Action d'un dérivé de l'acide benzoylacétique sur un alcoolate de magnésium, éventuellement préparé in situ, selon le schéma réactionnel:

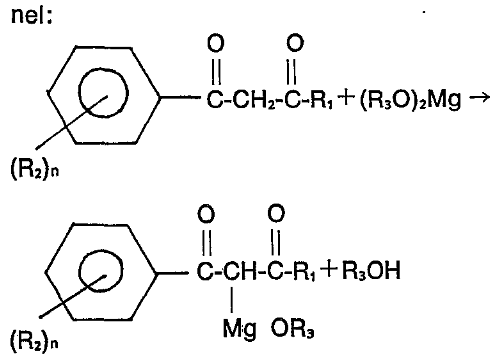

dans lequel $R_1$, $R_2$ et n ont la même signification que dans la revendication 1 et $R_3$ représente un radical alcoyle contenant de 1 à 4 atomes de carbone.

B - Action du chlorure d'un acide trihalogénoacrylique sur le dérivé alcoxymagnésien résultant de l'étape A, selon le schéma réactionnel:

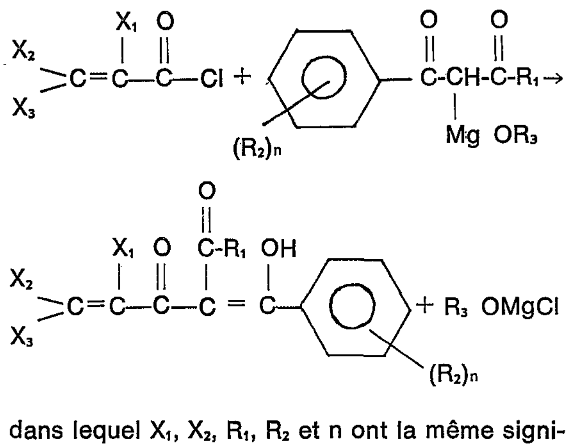

dans lequel $X_1$, $X_2$, $R_1$, $R_2$ et n ont la même signification que dans la revendication 1 et $X_3$ représente un atome d'halogène.

C - Cyclisation du composé énolique résultant de l'étape B.

7. Procédé selon la revendication 6, caractérisé en ce que l'étape A est effectuée en milieu anhydre, dans un solvant inerte à une température comprise entre 35 et 150°C.

8. Procédé selon la revendication 6, caractérisé en ce que l'étape B est effectuée en milieu anhydre, dans un solvant inerte, à une température comprise entre 0 et 40°C.

9. Procédé selon la revendication 6, caractérisé en ce que l'étape C est effectuée en traitant par une solution aqueuse diluée d'un acide fort, le mélange réactionnel résultant de l'étape B, à une température comprise entre 0 et 30°C, puis en séparant la phase organique de la phase aqueuse, et en chauffant ensuite la phase organique à une température comprise entre 30° et 160°C.

**Patentansprüche für die Vertragsstaaten:**
BE, CH, DE, FR, GB, IT, LU, NL, SE

1. 2-Phenyl-4-pyron-Verbindungen der allgemeinen Formel

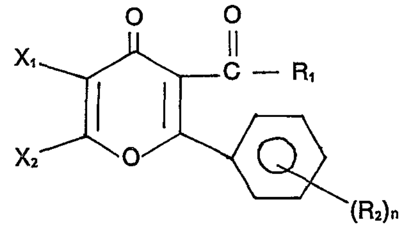

in der $X^1$ und $X^2$ gleich oder verschieden sind und jeweils für ein Wasserstoffatom oder ein Halogenatom stehen, mit der Massgabe, dass mindestens einer der Substituenten ein Halogenatom bedeutet,

$R^1$ für eine Alkyloxygruppe mit 1 bis 4 Kohlenstoffatomen, eine Alkenyloxygruppe mit 2 bis 4 Kohlenstoffatomen, die Propargyloxygruppe, eine Halogenalkyloxygruppe mit 1 bis 4 Kohlenstoffatomen, die Aminogruppe, eine Alkylaminogruppe mit 1 bis 4 Kohlenstoffatomen oder eine Dialkylaminogruppe mit jeweils 1 bis 4 Kohlenstoffatomen in den Alkylgruppen, die gleich oder verschieden sind, steht,

$R^2$ ein Halogenatom, eine Alkylgruppe mit 1 bis 5 Kohlenstoffatomen, eine Alkyloxygruppe mit 1 bis 4 Kohlenstoffatomen, eine Alkenyloxygruppe mit 3 oder 4 Kohlenstoffatomen, eine Alkinyloxygruppe mit 3 oder 4 Kohlenstoffatomen, eine Alkylthiogruppe mit 1 bis 4 Kohlenstoffatomen, eine Halogenalkylgruppe mit 1 bis 4 Kohlenstoffatomen, eine Halogenalkyloxygruppe mit 1 bis 4 Kohlenstoffatomen, eine Halogenalkylthiogruppe mit 1 bis 4 Kohlenstoffatomen, die Nitrogruppe, OH-Gruppe, CN-Gruppe oder die Benzyloxygruppe bedeutet und

n die Zahl 0, 1, 2, 3, 4 oder 5 ist mit der Massgabe, dass wenn $n \geqq 2$, die Substituenten $R^2$ gleich oder verschieden sein können.

2. Verbindung nach Anspruch 1 der allgemeinen Formel

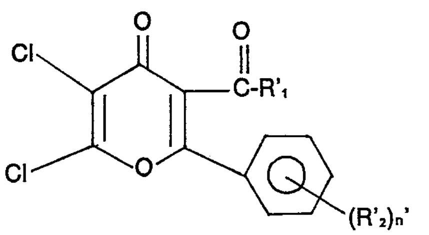

in der R'[1] eine Alkyloxygruppe mit 1 bis 4 Kohlenstoffatomen, die Allyloxygruppe, Propargyloxygruppe oder eine Dialkylaminogruppe mit jeweils 1 bis 4 Kohlenstoffatomen in den Alkylgruppen, die gleich oder verschieden sein können, ist,

R'[2] für ein Halogenatom, eine Alkylgruppe mit 1 bis 4 Kohlenstoffatomen, eine Alkyloxygruppe mit 1 bis 4 Kohlenstoffatomen, für die Allyloxygruppe, Propargyloxygruppe oder die Trifluormethylgruppe steht und

n' die Zahl 1, 2 oder 3 ist mit der Massgabe, dass wenn n' $\geqq$ 2, die Substituenten R'[2] gleich oder verschieden sein können.

3. 2-(4-Fluorphenyl)-3-äthoxycarbonyl-5,6-dichlorpyron-4 nach Anspruch 1.

4. 2-(3-Trifluormethylphenyl)-3-äthoxycarbonyl-5,6-dichlorpyron-4 nach Anspruch 1.

5. Verfahren zur Herstellung der Verbindungen nach Anspruch 1, dadurch gekennzeichnet, dass es folgende drei Stufen umfasst:

A) Einwirkung eines Derivats der Benzoylessigsäure auf ein gegebenenfalls in situ hergestelltes Magnesiumalkoholat entsprechend dem Reaktionsschema

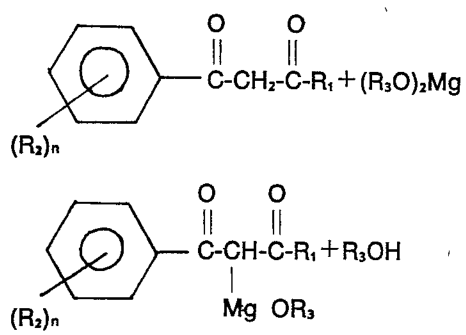

wobei R¹, R² und n die gleiche Bedeutung wie in Anspruch 1 haben und R³ für eine Alkylgruppe mit 1 bis 4 Kohlenstoffatomen steht,

B) Einwirkung des Chlorids einer Trihalogenacrylsäure auf die in Stufe A erhaltene Alkoxymagnesiumverbindung entsprechend dem Reaktionsschema

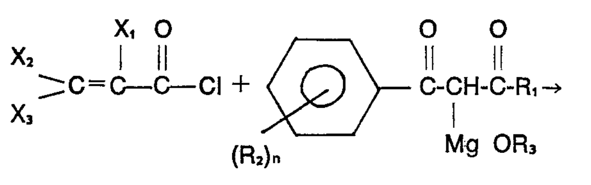

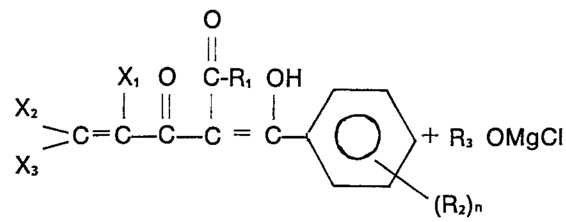

wobei X¹, X², R¹, R² und n die gleiche Bedeutung wie in Anspruch 1 haben und X³ für ein Halogenatom steht,

C) Cyclisierung der in Stufe 2 erhaltenen Enolverbindung zur angestrebten Verbindung der in Anspruch 1 angegebenen Formel.

6. Verfahren nach Anspruch 5, dadurch gekennzeichnet, dass man die Stufe A in wasserfreiem Medium in einem inerten Lösungsmittel bei einer Temperatur von 35 bis 150°C ausführt.

7. Verfahren nach Anspruch 5, dadurch gekennzeichnet, dass man die Stufe B in wasserfreiem Medium in einem inerten Lösungsmittel bei einer Temperatur von 0 bis 40°C ausführt.

8. Verfahren nach Anspruch 5, dadurch gekennzeichnet, dass man die Stufe C ausführt, indem man das in Stufe B erhaltene Reaktionsgemisch mit einer verdünnten wässrigen Lösung einer starken Säure bei einer Temperatur von 0 bis 30°C behandelt, die organische Schicht von der wässrigen Schicht trennt und schliesslich die organische Schicht auf eine Temperatur von 30 bis 160°C erhitzt.

9. Mittel zur Bekämpfung von Pilzerkrankungen bei Pflanzen, dadurch gekennzeichnet, dass es eine wirksame Menge mindestens einer Verbindung nach Anspruch 1 enthält, zusammen mit einem in der Landwirtschaft gebräuchlichen und mit dem Wirkstoff verträglichen Träger und/oder grenzflächenaktiven Mittel.

10. Mittel nach Anspruch 9, dadurch gekennzeichnet, dass es 0,01 bis 95 Gew.-% Wirkstoff enthält.

11. Verfahren zur Behandlung von Pflanzen gegen Pilzerkrankungen, dadurch gekennzeichnet, dass man ein Mittel nach einem der Ansprüche 9 oder 10 anwendet.

**Patentansprüche für den Vertragsstaat:** AT

1. Fungizides Mittel zur Bekämpfung von Pilzerkrankungen bei Pflanzen, dadurch gekennzeichnet, dass es, als Wirkstoff, mindestens eine Verbindung der allgemeinen Formel:

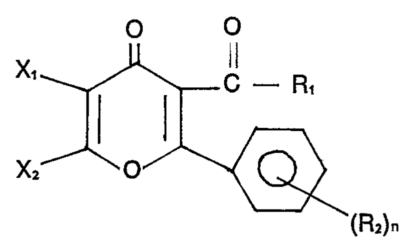

enthält, in welcher:

X¹ und X² gleich oder verschieden sind und

jeweils für ein Wasserstoffatom oder ein Halogenatom stehen, mit der Massgabe, dass mindestens einer der Substituenten ein Halogenatom bedeutet,

$R^1$ eine Alkyloxygruppe mit 1 bis 4 Kohlenstoffatomen, eine Alkenyloxygruppe mit 2 bis 4 Kohlenstoffatomen, die Propargyloxygruppe, eine Halogenalkyloxygruppe mit 1 bis 4 Kohlenstoffatomen, die Aminogruppe, eine Alkylaminogruppe mit 1 bis 4 Kohlenstoffatomen oder eine Dialkylaminogruppe mit jeweils 1 bis 4 Kohlenstoffatomen in den Alkylgruppen, die gleich oder verschieden sind, bedeutet,

$R^2$ ein Halogenatom, eine Alkylgruppe mit 1 bis 5 Kohlenstoffatomen, eine Alkyloxygruppe mit 1 bis 4 Kohlenstoffatomen, eine Alkenyloxygruppe mit 3 oder 4 Kohlenstoffatomen, eine Alkinyloxygruppe mit 3 oder 4 Kohlenstoffatomen, eine Alkylthiogruppe mit 1 bis 4 Kohlenstoffatomen, eine Halogenalkylgruppe mit 1 bis 4 Kohlenstoffatomen, eine Halogenalkyloxygruppe mit 1 bis 4 Kohlenstoffatomen, eine Halogenalkylthiogruppe mit 1 bis 4 Kohlenstoffatomen, die Nitrogruppe, Hydroxy-Gruppe, Cyano-Gruppe oder die Benzyloxygruppe bedeutet und

n die Zahl 0, 1, 2, 3, 4 oder 5 ist mit der Massgabe, dass wenn $n \geqq 2$, die Substituenten $R^2$ gleich oder verschieden sein können.

2. Mittel nach Anspruch 1, dadurch gekennzeichnet, dass es, als Wirkstoff, mindestens eine Verbindung der Formel:

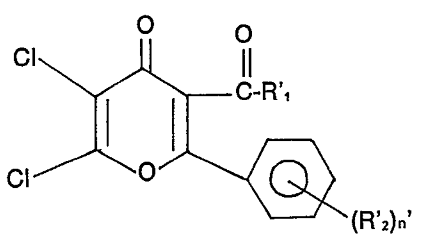

enthält, in welcher $R'^1$, eine Alkyloxygruppe mit 1 bis 4 Kohlenstoffatomen, die Allyloxygruppe, Propargyloxygruppe oder eine Dialkylaminogruppe mit jeweils 1 bis 4 Kohlenstoffatomen in den Alkylgruppen, die gleich oder verschieden sein können, bedeutet,

$R'^2$ ein Halogenatom, eine Alkylgruppe mit 1 bis 4 Kohlenstoffatomen, eine Alkyloxygruppe mit 1 bis 4 Kohlenstoffatomen, eine Allyloxygruppe, eine Propargyloxygruppe oder die Trifluormethylgruppe bedeutet und

n' die Zahl 1, 2 oder 3 ist mit der Massgabe, dass wenn $n' \geqq 2$, die Substituenten $R'^2$ gleich oder verschieden sein können.

3. Mittel nach Anspruch 1, dadurch gekennzeichnet, dass es, als Wirkstoff, mindestens eine Verbindung die unter 2-(4-Fluorphenyl)-3-äthoxycarbonyl-5,6-dichlor-pyron-4 und 2-(3-Trifluormethylphenyl)-3-äthoxycarbonyl-5,6-dichlor-pyron-4, ausgewählt ist, enthält.

4. Mittel nach Anspruch 1, dadurch gekennzeichnet, dass es 0,01 bis 95 Gew.-% Wirkstoff enthält, zusammen mit einem in der Landwirtschaft gebräuchlichen Träger und/oder grenzflächenatkiven Mittel.

5. Verfahren zur Behandlung von Pflanzen gegen Pilzerkrankungen, dadurch gekennzeichnet, dass man ein Mittel nach einem der Ansprüche 1 bis 4 anwendet.

6. Verfahren zur Herstellung einer Verbindung der allgemeinen Formel:

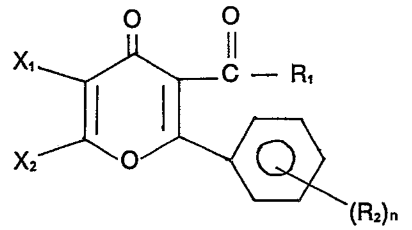

in welcher $X^1$, $X^2$, $R^1$, $R^2$ und n die gleiche Bedeutung wie in Anspruch 1 haben, dadurch gekennzeichnet, dass es folgende drei Stufen umfasst:

A) Einwirkung eines Derivats der Benzoylessigsäure auf ein gegebenenfalls in situ hergestelltes Magnesiumalkoholat entsprechend dem Reaktionsschema:

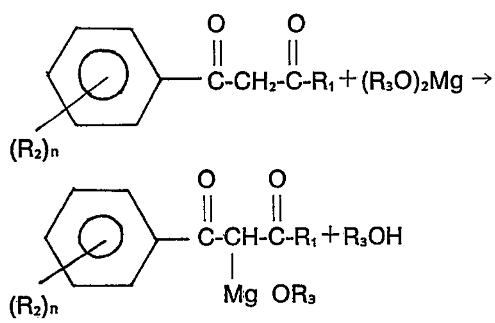

wobei $R^1$, $R^2$ und n die gleiche Bedeutung wie in Anspruch 1 haben und $R^3$ für eine Alkylgruppe mit 1 bis 4 Kohlenstoffatomen steht,

B) Einwirkung des Chlorids einer Trihalogenacrylsäure auf die in Stufe A erhaltene Alkoxymagnesiumverbindung entsprechend dem Reaktionsschema

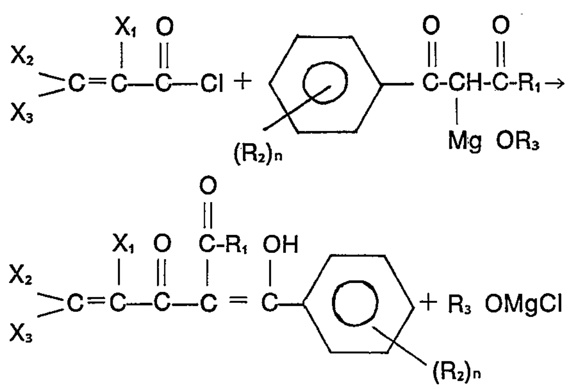

wobei $X^1$, $X^2$, $R^1$, $R^2$ und n die gleiche Bedeutung wie in Anspruch 1 haben und $X^3$ für ein Halogenatom steht,

C) Cyclisierung der in Stufe B erhaltenen Enolverbindung.

7. Verfahren nach Anspruch 6, dadurch gekennzeichnet, dass man die Stufe A in wasserfreiem Medium in einem inerten Lösungsmittel bei einer Temperatur von 35 bis 150°C ausführt.

8. Verfahren nach Anspruch 6, dadurch gekennkeichnet, dass man die Stufe B in wasserfreiem Medium in einem inerten Lösungsmittel bei einer Temperatur von 0 bis 40°C ausführt.

9. Verfahren nach Anspruch 6, dadurch gekennzeichnet, dass man die Stufe C ausführt, indem man das in Stufe B erhaltene Reaktionsgemisch mit einer verdünnten wässrigen Lösung einer starken Säure bei einer Temperatur von 0 bis 30°C behandelt, die organische Schicht von der wässrigen Schicht trennt und schliesslich die organische Schicht auf eine Temperatur von 30 bis 160°C erhitzt.

**Claims for the contracting states:**
BE, CH, DE, FR, GB, IT, LU, NL, SE

1. A 2-phenyl-4-pyrone derivative of the formula

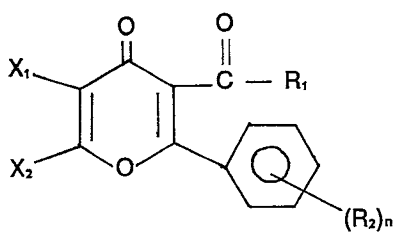

in which $X_1$ and $X_2$, which are identical or different, each represent a hydrogen or halogen atom, with the proviso that at least one of these substituents represents a halogen atom, $R_1$ represents an ankoxy radical containing from 1 to 4 carbon atoms, an alkenyloxy radical containing from 2 to 4 carbon atoms, the propargyloxy radical, a halogenalkoxy radical containing from 1 to 4 carbon atoms, an amino radical, an alkylamino radical containing from 1 to 4 carbon atoms or a dialkylamino radical in which the alkyl parts each of which comprising from 1 to 4 carbon atoms are identical or different, $R_2$ represents a halogen atom, an alkyl radical containing from 1 to 5 carbon atoms, an alkoxy radical containing from 1 to 4 carbon atoms, an alkenyloxy radical containing from 3 to 4 carbon atoms, an alkynyloxy radical containing from 3 to 4 carbon atoms, an alkylthio radical containing from 1 to 4 carbon atoms, a halogenoalkyl radical containing from 1 to 4 carbon atoms, a halogenalkoxy radical containing from 1 to 4 carbon atoms, a halogenalkylthio radical containing from 1 to 4 carbon atoms or a nitro, hydroxyl, cyano or benzyloxy radical and n is an integer which can be equal to 0, 1, 2, 3, 4 or 5, it being understood that, if n is greater than or equal to 2, the substituents $R_2$ can be identical or different.

2. A compound according to claim 1, of the formula:

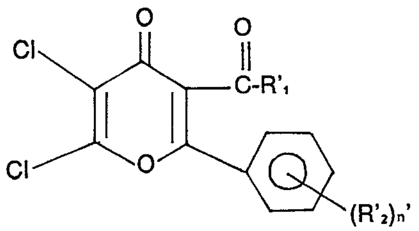

in which $R'_1$ represents an alkoxy radical containing from 1 to 4 carbon atoms, an allyloxy or propargyloxy radical or a dialkylamino radical in which each of the alkyl parts, which are identical or different, contains from 1 to 4 carbon atoms, $R'_2$ represents a halogen atom, an alkyl radical containing from 1 to 4 carbon atoms, an alkoxy radical containing from 1 to 4 carbon atoms, an allyloxy or propargyloxy radical or the trifluoromethyl radical and n' is an integer equal to 1, 2 or 3, it being understood that, if n' is greater than or equal to 2, the radicals $R'_2$ can be identical or different.

3. A compound according to claim 1, which is 2-(4-fluorophenyl)-3-ethoxycarbonyl-5,6-dichloro-4-pyrone.

4. A compound according to claim 1, which is 2-(3-trifluoromethylphenyl)-3-ethoxycarbonyl--5,6-dichloro-4-pyrone.

5. A process for the preparation of a compound according to claim 1, which comprises the following three steps:

A. Reaction of a benzoylacetic acid derivative with a magnesium alcoholate, optionally prepared in situ, in accordance with the equation:

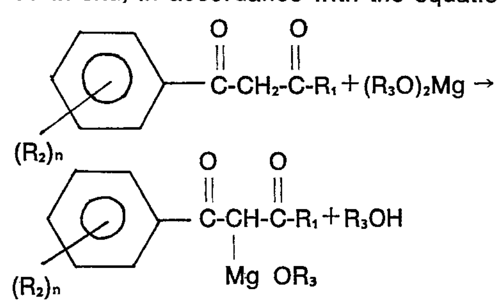

in which $R_1$, $R_2$ an n have the same meaning as in claim 1 and $R_3$ represents an alkyl radical containing from 1 to 4 carbon atoms.

B. Reaction of the chloride of a trihalogenoacrylic acid with the alkoxymagnesium derivative resulting from step A, in accordance with the equation

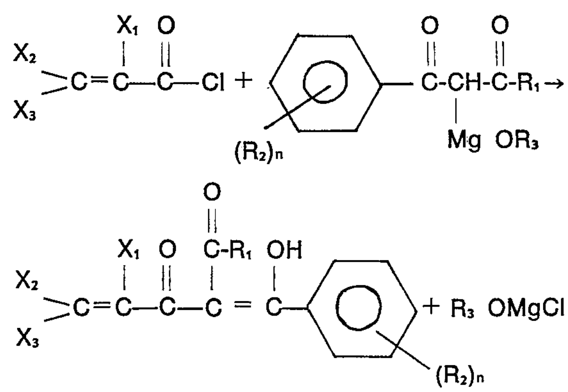

in which $X_1$, $X_2$, $R_1$, $R_2$ and n have the same meaning as in claim 1 and $X_3$ represents a halogen atom.

C. Cyclisation of the enolic compound resulting from step B, to give the compound according to claim 1.

6. A process according to claim 5, wherein step A is carried out in an anydrous medium, in an inert solvent, at a temperature between 35 and 150°C.

7. A process according to claim 5, wherein step B is carried out in an anhydrous medium, in an inert solvent, at a temperature between 0 and 40°C.

8. A process according to claim 5, wherein step C is carried out by treating the reaction mixture resulting from step B with a dilute aqueous solution of a strong acid, at a temperature between 0 and 30°C, by then separating the organic phase from the aqueous phase and by subsequently heating the organic phase at a temperature between 30° and 160°C.

9. A composition which can be used for protecting plants against fungal diseases, which contains an effective amount of at least one compound according to claim 1, in association with a carrier and/or a surface-active agent which can be used in agriculture and are compatible with the said active ingredient.

10. A composition according to claim 9, which contains from 0.01 to 95% by weight of active ingredient.

11. A process for treating plants against fungal diseases, which comprises using a composition according to one of claims 9 to 10.

**Claims for the contracting state:** AT

1. A fungicidal composition, for protecting plants against fungal diseases, which contains, as active ingredient, at least one compound of the general formula:

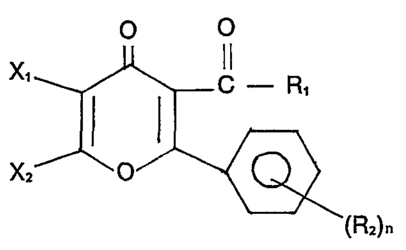

in which $X_1$ and $X_2$, which are identical or different, each represent a hydrogen or halogen atom, with the proviso that at least one of these substituents represents a halogen atom, $R_1$ represents an alkoxy radical containing from 1 to 4 carbon atoms, an alkenyloxy radical containing from 2 to 4 carbon atoms, the propargyloxy radical, a halogenalkoxy radical containing from 1 to 4 carbon atoms, an amino radical, an alkylamino radical containing from 1 to 4 carbon atoms or a dialkylamino radical in which the alkyl parts ($C_1$-$C_4$) are identical or different, $R_2$ represents a halogen atom, an alkyl radical containing from 1 to 5 carbon atoms, an alkoxy radical containing from 1 to 4 carbon atoms, an alkenyloxy radical containing from 3 to 4 carbon atoms, an alkynyloxy radical containing from 3 to 4 carbon atoms, an alkylthio radical containing from 1 to 4 carbon atoms, a halogenoalkyl radical containing from 1 to 4 carbon atoms, a halogenoalkoxy radical containing from 1 to 4 carbon atoms, a halogenalkylthio radical containing from 1 to 4 carbon atoms or a nitro, hydroxyl, cyano or benzyloxy radical and n is an integer which can be equal to 0, 1, 2, 3, 4 or 5, it being understood that, if n is greater than or equal to 2, the substituents $R_2$ can be identical or different.

2. A composition according to claim 1 which contains as active ingredient at least one compound of the formula:

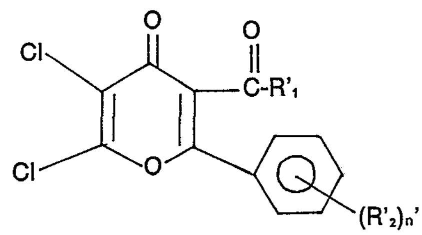

in which $R'_1$ represents an alkoxy radical containing from 1 to 4 carbon atoms, an allyloxy or propargyloxy radical or a dialkylamino radical in which each of the alkyl parts, which are identical or different, contains from 1 to 4 carbon atoms, $R'_2$ represents a halogen atom, an alkyl radical containing from 1 to 4 carbon atoms, an alkoxy radical containing from 1 to 4 carbon atoms, an allyloxy or propargyloxy radical or the trifluoromethyl radical and n' is an integer equal to 1, 2 or 3, it being understood that, if n' is greather than or equal to 2, the radicals $R'_2$ can be identical or different.

3. A composition according to claim 1 which contains as active ingredient at least one compound selected from amongst the 2-(4-fluorophenyl)-3-ethoxycarbonyl-5,6-dichloro-4-pyrone and the 2-(3-trifluormethylphenyl)-3-ethoxycarbonyl-5,6-dichloro-4-pyrone.

4. A composition according to claim 1 which contains from 0.01 to 95% by weight of active ingredient in association with a carrier and/or a surface-active agent which can be used in agriculture.

5. A process for treating plants against fungal diseases, which comprises using a composition according to one of claims 1 to 4.

6. A process for the preparation of a compound of the formula:

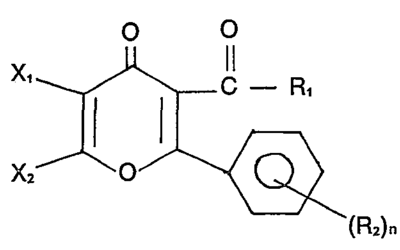

in which $X_1$, $X_2$, $R_1$, $R_2$ and n have the same meaning as in claim 1, which comprises the following three steps:

A. Reaction of a benzoylacetic acid derivative with a magnesium alcoholate, optionally prepared in situ, in accordance with the equation:

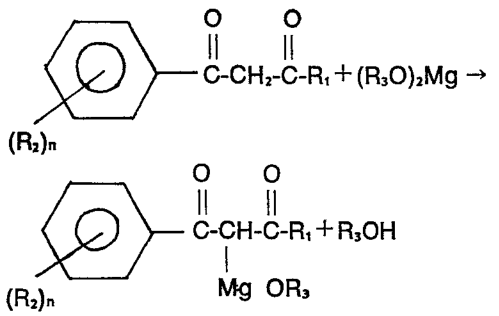

in which $R_1$, $R_2$ and n have the same meaning as in claim 1 and $R_3$ represents an alkyl radical containing from 1 to 4 carbon atoms.

B. Reaction of the chloride of a trihalogenoacrylic acid with the alkoxymagnesium derivative resulting from step A, in accordance with the equation

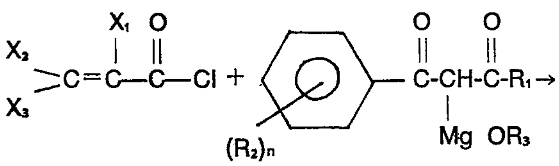

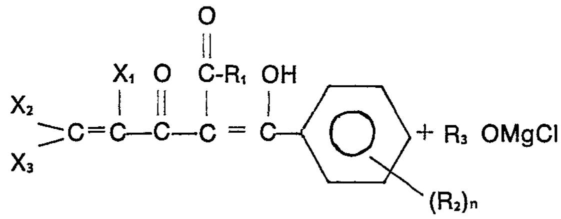

in which $X_1$, $X_2$, $R_1$, $R_2$ and n have same meaning as in claim 1 and $X_3$ represents a halogen atom.

C. Cyclisation of the enolic compound resulting from step B.

7. A process according to claim 6, wherein step A is carried out in an anydrous medium, in an inert solvent, at a temperature between 35 and 150°C.

8. A process according to claim 6, wherein step B is carried out in an anhydrous medium, in an inert solvent, at a temperature between 0 and 40°C.

9. A process according to claim 6, wherein step C is carried out by treating the reaction mixture resulting from step B with a dilute aqueous solution of a strong acid, at a temperature between 0 and 30°C, by then separating the organic phase from the aqueous phase and by subsequently heating the organic phase at a temperature between 30° and 160°C.